# EUROPEAN PATENT SPECIFICATION

(11) **EP 4 277 890 B1**
(45) Date of publication and mention of the grant of the patent: **08.10.2025**
(21) Application number: 22700282.1
(22) Date of filing: 03.01.2022
(51) Int. Cl.: C07C 227/02, C07C 227/18, C07C 229/16, C07C 229/12, C11D 3/33

(54) **PROCESS FOR MANUFACTURING CARBOXYLIC ACIDS**
VERFAHREN ZUR HERSTELLUNG VON CARBOXYLSÄUREN
PROCÉDÉ DE FABRICATION D'ACIDES CARBOXYLIQUES

(30) Priority: 15.01.2021 EP 21151770
(43) Date of publication of application: 22.11.2023
(73) Proprietor: BASF SE, 67056 Ludwigshafen am Rhein (DE)
(72) Inventor: TELES, Joaquim Henrique, 67056 Ludwigshafen (DE); HUELLMANN, Michael, 67056 Ludwigshafen (DE); GUTHERTZ, Alexandre, 67056 Ludwigshafen (DE); VAUTRAVERS, Nicolas, 67056 Ludwigshafen (DE); DODEKATOS, Georgios, 67056 Ludwigshafen (DE); REINOSO GARCIA, Marta, 67056 Ludwigshafen (DE)
(74) Representative: BASF IP Association
(86) International application number: PCT/EP2022/050029
(87) International publication number: WO 2022/152588

(56) References cited:
- WO-A1-2012/159952
- JP-A- 2000 063 338
- JP-A- S5 377 009
- US-A- 6 046 356

## Description

The present invention is directed towards a process for the synthesis of compounds with the general formula (II) or their respective alkali metal salts by oxidation of compounds of general formula (I) with oxygen 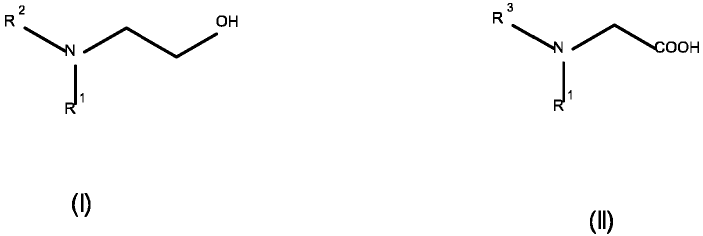
in the presence of a heterogeneous catalyst containing a metal of group 10 of the periodic table of the elements,
wherein R¹ and R² and R³ are same or different and selected from H and C₁-C₆-alkyl, non-substituted or substituted with one or more moieties selected from COOH and O-R⁴ with R⁴ being selected from H and C₁-C₄-alkyl, wherein the catalytically active metal is preferably Pt and wherein, preferably, at least one promotor selected from Cd, TI, Pb and Bi is present in the heterogeneous catalyst, and wherein the oxidation is, preferably, carried out at a temperature in the range of from -10 to +40°C.

The manufacture of N-bearing substituted carboxylic acids is still a topic of general interest. Although many of the basic N-bearing alcohols are readily available the resulting carboxylic acids that may be obtained bear impurities that are not accepted or at least undesired.

Modern cleaning compositions need to meet many requirements. They need to work under various conditions, for example various temperatures. They need to yield excellent results, and in the case of hard surface cleaners and in particular of automatic dishwashing formulations, they need to provide excellent results with respect to spotting and filming. In case glass is to be cleaned, glass corrosion needs to be inhibited or at least strongly reduced. Cleaning compositions need to be environmentally friendly, and they have to work even under conditions where only so-called "hard water" is available, for example water with a comparatively high content of Mg²⁺ and Ca²⁺ salts.

In WO 94/29421, chelating agents are disclosed that are environmentally friendly. However, the manufacturing process disclosed in WO 94/29421 does not exclude certain undesired impurities, for example nitrilotriacetate ("NTA"), and it requires the use of hydrocyanic acid or cyanides.

In WO 2012/159952, a process for the manufacture of certain N-bearing carboxylic acids is disclosed that avoids the formation of NTA. The alkoxylation step is convenient and followed by a catalytic oxidative dehydrogenation in the presence of alkali metal hydroxide. However, the alkoxylation step may lead to the formation of ethylene glycol that is subsequently easily converted to oxalic acid. Oxalates are undesired in many of the subsequent applications of carboxylic acids, especially in automatic dishwashing applications.

JP53077009A (JPA1978077009) refers to a process for manufacturing an aminocarboxylic acid, using an (undoted) platinum catalyst on a support.

JP 2000 063338 A discloses a process for manufacturing sodium N-methylglycine (sodium sarcosinate), using gold (Au) as active metal.

US 6046356 describes a process for preparing aqueous solutions of betaines; the experimental evidence is restricted to the use of palladium or platinum on a support as active metals at temperatures in the range from 70° to 80° C.

It was therefore the objective of the present invention to provide N-bearing carboxylic acids that do not contain the above discussed undesired impurities. It was further an objective to provide a process that avoids the disadvantages described above. Furthermore, it was an objective to provide N-bearing carboxylic acids with sufficient purity.

Accordingly, the process defined at the outset has been found, hereinafter also defined as inventive process or as process according to the present invention.

The inventive process starts off from a compound according to general formula (I) 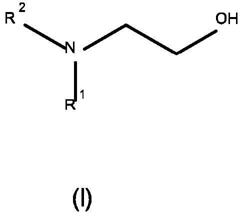
wherein R¹ and R² are same or preferably different and selected from
H and
C₁-C₆-alkyl, non-substituted or preferably substituted with one or more moieties selected from COOH and O-R⁴, with R⁴ being selected from H and C₁-C₄-alkyl, preferably H.

Examples of C₁-C₆-alkyl are methyl, ethyl, n-propyl, iso-propyl, n-butyl, iso-butyl, sec.-butyl, tert.-butyl, n-pentyl, iso-pentyl, sec.-pentyl, neo-pentyl, 1,2-dimethylpropyl, iso-amyl, n-hexyl, iso-hexyl, and sec.-hexyl, preferred are C₂-C₅-alkyl.

Preferably, at least one of R¹ and R² is C₂-C₅-alkyl that is substituted with at least one moiety, for example with a hydroxyl group that may be a secondary or tertiary or preferably a primary hydroxy group. Particularly, R² is selected from (CH₂)₂-OH, (CH₂)₃-OH and (CH₂)₃-OH, more preferred is -CH₂-CH₂-OH.

Preferably, R¹ is selected from C₁-C₆-alkyl that is substituted with one or two COOH groups. Particularly, R¹ is selected from -CHR⁵R⁶ with R⁵ and R⁶ being same or preferably different and selected from H and C₁-C₆-alkyl that is substituted with one or two COOH groups wherein R⁵ and R⁶ together bear no more than 5 C-atoms other than those that are part of a COOH group.

More preferably, R¹ is CH₃-CH(COOH) or (CH₃)₂CH-CH₂-CH-COOH or (CH₃)₂CH-CH-COOH or C₂H₅-CH(CH₃)-CH-COOH or HO-CH₂-CH-COOH or HOOC-(CH₂)₂-CH-COOH, as free acid or at least partially neutralized with alkali, for example with sodium, and even more preferably, R¹ is CH₃-CH(COOH) or HOOC-(CH₂)₂-CH-COOH, as free acid or at least partially neutralized with alkali, for example fully neutralized with sodium.

In one embodiment of the present invention, compound (I) is selected from compounds of general formula (I a) 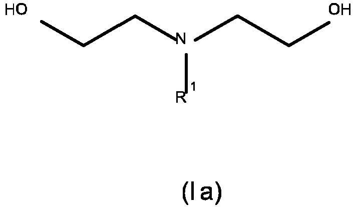 and R¹ is defined as above.

More preferably, compound (I) is selected from compounds of general formula (I a) and R¹ is CH₃-CH(COOH) or (CH₃)₂CH-CH₂-CH-COOH or (CH₃)₂CH-CH-COOH or C₂H₅-CH(CH₃)-CH-COOH or HO-CH₂-CH-COOH or HOOC-(CH₂)₂-CH-COOH, and even more preferably, R¹ is CH₃-CH(COOH) or HOOC-(CH₂)₂-CH-COOH.

In embodiments wherein R¹ is chiral, compound (I) may be selected from the L-enantiomer and the D-enantiomer and an enantiomerically enriched mixture with predominantly the L-enantiomer, for example with an enantiomeric excess (ee) from 10 to 95%. In other embodiments, in such embodiments compound (I) is the racemic mixture. The enantiomeric excess may be determined by polarographic measurements or chromatographically, for example by HPLC spectroscopy.

Many compounds according to general formula (I) are known from the art, and they may be obtained by ethoxylation of compounds of general formula R¹R²N-H. Compounds according to general formula (I a) may be made by mono-ethoxylation of amines according to formula HO-CH₂CH₂-NH-R¹ or by two-fold ethoxylation of compounds of general formula R¹-NH₂. Preferably, compounds of general formula R¹-NH₂ are provided as bis-alkoxylated compounds with as low a percentage of monoalkoxylation by-product as possible, for example less than 1 mol-%.

Compounds according to general formula (I) and preferably (I a) may be introduced to the inventive process in bulk or preferably in aqueous medium. In one embodiment of the present invention, compounds according to formula (I) and especially of formula (I a) are purified. In another embodiment, they contain impurities stemming from their manufacture, and especially when they are introduced in an aqueous medium, they may contain ethylene glycol that stems from hydrolysis of unreacted ethylene oxide.

Especially when compounds according to formula (I) and particularly (I a) are made by ethoxylation with ethylene oxide they may bears an impurity of from 0.01 to 10% by weight of glycols, referring to compound (I), preferably 1 to 5% by weight. Examples of such impurities are ethylene glycol and diethylene glycol. Usually, compounds according to formula (I) and particularly (I a) bear more ethylene glycol than diethylene glycol as impurities.

The inventive process itself may be carried out with compound according to general formula (I) and especially (I a) being in aqueous medium, especially in an aqueous solution. Said aqueous medium may have a pH value in the range of 10 to 14, preferably 12 to 14. In one embodiment of the present invention, said aqueous medium contains at least 0.9 mol of a base per mol of hydroxyl group in compound of general formula (I), for example 0.9 to 1.5 mol, preferably 0.9 to 1.1 mol.

Examples of suitable bases are alkali metal carbonates and alkali metal hydroxides, for example Na₂CO₃, K₂CO₃, preferably potassium hydroxide and sodium hydroxide and mixtures thereof. In this context, compound of general formula (I) is not considered a base.

Aqueous medium in the context of the present invention is not restricted to pure water but may include a medium that is liquid at ambient temperature and that may contain one or more solvents that are miscible with water, for example methanol, ethanol, isopropanol, propylene glycol, or ethylene glycol, toluene, THF, 1,4-dioxane, and the like, with at least 50 vol-% of the total solvent being water. It is preferred, though, to not add any organic solvent that is oxidized in the course of the inventive process, for example to not add ethylene glycol. It is more preferred to not add any organic solvent and to use water as the sole solvent.

In one embodiment of the present invention, wherein the oxidation is carried out at a temperature in the range of from -10 to +40°C, preferably zero to 35°C, even more preferably 2 to 20°C.

The inventive process is carried out on the presence of a heterogeneous catalyst containing a metal of group 10 of the periodic table of the elements, for example nickel (Ni), palladium (Pd) or platinum (Pt) or combinations thereof, preferred is Pt. Said heterogeneous catalyst contains the metal of group 10 preferably in the oxidation state of zero, for example Pt⁰. Preferably, said heterogeneous catalyst is used in the form of a finely dispersed metal powder or as sponge-metal catalyst or as skeletal catalyst.

Even more preferably, said metal of group 10 of the periodic table of the elements is deposited on a support. Suitable supports are silica, zirconia, titania, alumina and especially carbon, for example as charcoal.

Said supports can vary in specific area ("BET surface") from 1 to 600 m² g⁻¹. The BET surface may be determined by nitrogen adsorption in accordance with DIN ISO 9277:2010.

Said support may contain impurities that do not impede the reaction. Carbon supports used for this invention contain impurities, such as K, Mg, Ca, Al, Si, P, S, Cl, Br, Ti, Zr, Cr, Mn, Fe, Cu and combinations of at least two of the aforementioned.

Said support may be present in different shapes, such as tables, pellets, split or extrudates. Preferably, said support is used as powder with an average particle diameter (D50) in the range of from 1 µm to 1 mm.

The catalyst loading on the support may vary from 0.1 to 10 wt.-%. Preferably, the catalyst loading is from 1 to 5 wt.-%. Most preferably, the catalyst loading is from 4 to 5 wt.-%.

The catalytic activity of said metal of group 10 of the periodic table of the elements may be enhanced by a promotor. Promotors are usually in metallic or oxide form and in a molar ratio in the range of from metal/promotor from 10:1 to 1:4 metal/promotor with respect to the metal(s) of group 10 of the periodic table of the element.

In one embodiment of the present invention, at least one promotor selected from Cd, Tl, Pb and Bi is present in the heterogeneous catalyst. For toxicity reasons, Bi is preferred.

In embodiments wherein said metal of group 10 of the periodic table of the elements is deposited on a support it is preferred to also deposit a promotor on said support.

Catalysts like Pt or Pd supported on charcoal are commercially available.

In embodiments where the application of a promotor is desired a supported catalyst of a metal of group 10 of the periodic table of the elements may be impregnated with an aqueous or alcoholic solution of a compound of said promotor followed by removal of the water or alcohol, as the case may be, and reduction activation, for example with hydrogen or alkali metal formate, for example sodium formate. Suitable compounds of said promotor are the nitrates.

In other embodiments, the support is impregnated with an alcoholic or aqueous solution of compounds of both the metal(s) of group 10 of the periodic table of the elements and the promotor, for example with an aqueous solution of platinum nitrate and bismuth nitrate.

The inventive process is carried out in an atmosphere that contains oxygen. Examples are pure oxygen, air, oxygen-enriched air, "synthetic air" made by mixing oxygen and nitrogen in a molar ratio of 1:4, and air diluted with nitrogen, for example in a ratio of 1:100 to 1:3.9. However, the inventive process may be performed under an atmosphere of oxygen-enriched air as well, for example in a molar ratio of oxygen : nitrogen of 100 : 1 to 1 : 4.1.

The inventive process may be carried out at reduced pressure, for example 50 mbar to 995 mbar (abs), or under ambient pressure or at elevated pressure, for example at a pressure in the range of from 1.05 to 10 atm (abs). In a preferred embodiment, oxygen content and pressure are adjusted in a way that the partial pressure of oxygen (O₂) during the oxidation is in the range of from 0.05 to 8 bar.

In one embodiment of the present invention, the duration of the inventive process is in the range of from 30 minutes to 3 days, preferably 2 hours to 12 hours.

The inventive process may be carried out in various types of vessels that allow good contact of a liquid and a gas. Examples may be found in Perry's Chemical Engineers' Handbook, 8th Edition, Editor: Don W. Green, McGraw Hill, 2008, especially section "Multiphase Reactors" with pages 19-53 to 19-61. Examples are agitated slurry reactors, slurry bubble-column reactors and in particular jet loop reactors, fluidized gas-liquid-solid reactors, trickle bed reactors, and packed bubble columns.

In one embodiment of the present invention, stirred tank reactors are suitable, especially when they are equipped with a gassing stirrer. Particularly useful gassing stirrers are turbine gassing stirrers such as Rushton turbine stirrers. On laboratory scale, it is preferred to introduce the oxygen through a glass frit.

The inventive process may be performed as batch reaction or in continuous form.

In one embodiment of the present invention, the reaction product of the inventive process essentially comprises a mixture of compounds according to general formula (II a) or (II b) 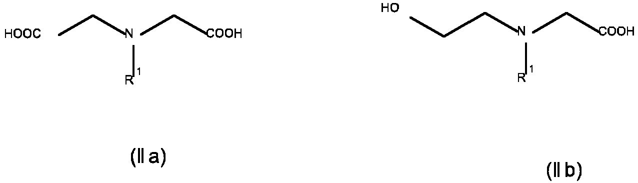 or their respective alkali metal salts wherein R¹ is defined as above. In this context, "essentially comprises" means that at least 80% by weight, preferably at least 85% by weight of the reaction product is selected from compounds according to general formula (II a) and (II b). In this context, water is thus neglected as well as solvents other than water, if applicable.

The composition of the reaction product of the inventive process may be determined by ¹H-NMR spectroscopy.

In one embodiment of the present invention, the reaction product essentially comprises compounds according to general formulae (II a) and (II b) in a molar ratio in the range of from 20:1 to 1:1, preferably 20:1 to 5:1, and in the range of in the range of from 2 to 15% by weight glycolic acid, preferably from 2 to 6 % by weight. Preferably, the amount of oxalic acid is in the range of from zero to 0.1% by weight. Even more preferably, no detectable amounts of oxalic acid are comprised.

The reaction product is usually alkaline, the percentages are in each case refer to the respective sodium salts, with all carboxylic acid groups being fully neutralized.

The inventive process may comprise further - optional - steps, for example work-up steps such as, but not limited to removal of the catalyst, bleaching or filtration over charcoal or silica. It is preferred to remove the catalyst, for example by filtration, by the way of a centrifuge, or by installing the catalyst in a reactor in which the respective reaction mixture is passed by the catalyst.

In addition, drying steps are possible, such as spray drying, spray granulation, or crystallization.

By carrying out the inventive process, compounds according to formula (II) may be obtained that contain only very small amounts of undesired impurities such as, but not limited to NTA and oxalic acid or the respective oxalates, in most cases even below detection level as shown by HPLC. Compounds according to formula (II) made by the inventive process are excellently suitable for applications in household, for example as builder for automatic dishwashing formulations.

A further aspect of the present invention is related to mixtures, hereinafter also referred to as inventive mixtures. Inventive mixtures comprise compounds according to general formulae (II a) and (II b) 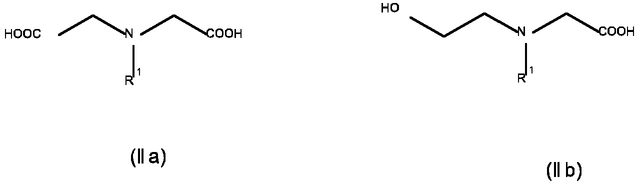
or their respective alkali metal salts in a molar ratio in the range of from 20:1 to 1:1, preferably 20:1 to 5:1 and in the range of from 2 to 15% by weight glycolic acid, preferably from 2 to 6 % by weight referring to the sum of compounds according to general formulae (II a) and (II b), and less than 0.1% by weight of oxalic acid, or their respective alkali metal salts,
wherein R¹ is selected from H and C₁-C₆-alkyl, non-substituted or substituted with one or more moieties selected from COOH and O-R⁴, with R⁴ being selected from H and C₁-C₄-alkyl.
R¹ and R³ are defined in more detail above.

In preferred inventive mixtures, R¹ is CH₃-CH(COOH) or HOOC-(CH₂)₂-CH-COOH, optionally partially or fully neutralized with alkali.

In one embodiment of the present invention, the molar ratio of compound according to general formulae (II a) and (II b) is in the range of from 20:1 to 1:1. The molar ratio in each case refers to the free acids.

In one embodiment of the present invention, inventive mixtures may contain minor amounts of cations other than alkali metal. It is thus possible that minor amounts, such as 0.001 to 0.5 mol-% of total chelating agent in said inventive salt, based on anion, bear alkali earth metal cations such as Mg²⁺ or Ca²⁺, or transition metal ions such as Fe²⁺ or Fe³⁺ cations. Such minor amounts shall be neglected in the context of the present invention.

In embodiments wherein R¹ is chiral, compound (II) may be selected from the L-enantiomer and the D-enantiomer and an enantiomerically enriched mixture with predominantly the L-enantiomer, for example with an enantiomeric excess (ee) from 10 to 95%. In other embodiments, in such embodiments compound (II) is the racemic mixture. The enantiomeric excess may be determined by polarographic measurements or chromatographically, for example by HPLC spectroscopy with a chiral stationary phase.

Finally, in general, the process is preferably conducted in a way that the reaction components and phases (including gas, liquid and catalyst) are well mixed so that the reaction is not limited by mass-transport phenomena.

The invention will be further illustrated by the following working examples.

### General:

All oxidation reactions were performed in an double-walled glass reactor with inner temperature control, paddle stirrer with 4 blades and a submerged gas inlet tube (glass frit). Temperature control of the reaction is achieved by circulating a cooling medium (water/glycol mixture) through the reactor mantle.

NL: norm liter, liter at 1 bar and 20°C as recommended in the ISO norm NF E49-301*NF ISO 8778 of 2018-11-17

### I. Ethoxylation

### I.1 Manufacture of a compound (I a.1), R¹ = CH₃-CH(COOH), neutralized with Na

251.9 g (2.83 mol) of L-alanine were slurried in 152 g of water, and 228.9 g (2.83 mol) of 49,4% by mass sodium hydroxide solution were added. The resulting mixture was introduced into a one-liter autoclave (material: V2A-Büchi reactor) and, after appropriate inertization, pressured with 2 bar of nitrogen. Subsequently, 274 g (6.22 mol) of ethylene oxide were metered in at 20-25°C within 8 hours and stirring was continued at this temperature for a further 4 hours.

After the removal of unconverted ethylene oxide, the autoclave was emptied. In this way, 907 g of aqueous reaction mixture were obtained as a clear colorless viscous solution. Compound (I a.1) was obtained. It bore 4.6% by weight of ethylene glycol and 0.3% by weight of diethylene glycol.

### II. Oxidation

As catalyst, Pt/C (activated charcoal supported Pt catalyst), commercially available catalyst with 5 wt.-% Pt loading, was chosen with Bi as promotor.

The commercially available Pt/C catalyst was impregnated with an aqueous solution of Bi(NO₃)₃ over a time of 30 minutes The Pt/Bi molar ratio was 4:1. An intermediate was obtained that was separated from supernatant liquid phase by filtration and subjected to reduction without further intermediate steps. Aqueous sodium formate solution (4 mole/I) was used as reducing agent, molar ratio formate: Bi of 7.5, at a temperature of 60°C over a time of 30 minutes. A catalyst was obtained that was removed from the water, washed with distilled water and dried.

### II.1 Oxidation of compound (I a.1) to MGDA-Na₃

Reactor 1 was charged with the of Pt,Bi/C (17.16g, 1.38 mmol, 4.08 wt.-% Pt (dry), 35.07 wt.-% in H₂O, molar ratio Pt:Bi was 3.57:1) in water (161 mL), and cooled to -2°C. Then, an aqueous solution of the sodium salt of (I a.1) (17.19 g, 55.2 mmol, 64 wt.-% in H₂O) was added. Sodium hydroxide (9.72 g, 121.5 mmol, 2.2eq, 50 wt.-% in H₂O) was then added within 10 minutes. The inner temperature control was set to 5°C. At 5°C, oxygen (20 NL/h) was bubbled through the reaction mixture using a glass frit. The mixture was stirred using a mechanical stirrer. After 5 hours, the oxygen flow was stopped, and the catalyst was removed by filtration. Full conversion of compound (I a.1) was observed (¹H NMR). The molar ratio of (II a.1) and (II b.1) (in each case, R¹ = CH₃-CH(COOH) was 19:1 (determined by ¹H NMR). Further impurities were 1% by weight of sodium acetate and sodium salt of glycolic acid, 2% by weight, both regarding to the sum of (II. a.1) and (II b.1) (determined by HPLC).

### Comparative examples

### II.1.2 Oxidation of compound (I a.1) to MGDA-Na₃ (Comparative example)

Example II. 1.1 was repeated, except that the inner temperature control was set to 70°C. After a reaction time of 5 hours full conversion of the compound I a.1 was observed (as determined by ¹H NMR). No traces of (II a.1) and (II b.1) was observed (determined by ¹H NMR). The following products could be identified, but were not quantified: Oxalic acid, glycolic acid, alanine-*N-*monoacetic acid, *N*-ethanolglycine, acetic acid.

### II.1.3 Oxidation of compound (I a.1) to MGDA-Na₃ (Comparative example)

Example II. 1.1 was repeated, except that the Pt,Bi/C was replace with Pt/C (11.2 g, 1.38 mmol, 5 wt.-% Pt (dry), 47.6 wt.-% in H₂O). After a reaction time of 24 hours no conversion of the compound I a.1 was observed (determined by ¹H NMR).

### II.1.4 Oxidation of compound (I a.1) to MGDA-Na₃ (Comparative example)

Example II. 1.1 was repeated, except that the Pt,Bi/C was replace with Pt/C (11.2 g, 1.38 mmol, 5 wt.-% Pt (dry basis), 47.6 wt.-% in H₂O) and the inner temperature control was set to 70°C. After a reaction time of 7 hours no conversion of the compound I a.1 was observed (determined by ¹H NMR).

It can be seen that the choice of catalyst (and its doting) and temperature play a crucial role in the manufacturing process.

### II.2 Oxidation of N-Methylethanolamine, (I.2) to sodium sarcosinate, (II.2)

Reactor 1 was charged with a suspension of Pt,Pb,Cd/C (6.59g, 1.32 mmol, 10 wt.-% Pt (dry), molar ratio Pt:Pb:C 6.5:5:1, 42 wt.-% in H₂O) in water (400 mL), corresponding to 2.5 mole-% Pt with respect to (I.2). At a temperature of 12°C, (N-methylethanolamine (4 g, 53 mmol, 1eq, R¹ = CH₃, R² = H (I.2)) was added. Sodium hydroxide (4.69 g, 59 mmol, 1.1eq, 50 wt.-% in H₂O) was then added over a period of 10 minutes. The inner temperature control was set to 20°C. At 20°C, oxygen (20 NL/h) was bubbled in the reaction through a glass frit. After 52 hours, the oxygen flow was stopped, and the catalyst was removed by filtration. A conversion of 96% of the N-methylethanolamine was observed by ¹H NMR. Sodium sarcosinate (II.2) was obtained with a selectivity above 90% (¹H NMR).

### II.3 Oxidation of N-Methyldiethanolamine to N-methyl-N,N-diacetic acid ("Na₂-MIDA")

Reactor 1 was charged with a suspension of Pt,Bi/C (32.63g, 4.2mmol, 2.5 mol%, 4 wt.-% Pt (dry), 62.3 wt.-% in H₂O, molar ratio, Pt : Bi = 3.57 : 1) in water (380 mL). At a temperature of 10°C, N-methyldiethanolamine (20 g, 168 mmol, 1 eq, (I a.3) with R¹ = CH₃) was added. Sodium hydroxide (29.54 g, 369 mmol, 2.2 eq, 50 wt.-% in H₂O) was then added over a period of 10 minutes. The inner temperature control was set to 20°C. At 20°C, oxygen (20 NL/h) was bubbled in the reaction through a glass frit. After 30 minutes, the oxygen flow was stopped, and the catalyst was removed by filtration. Full conversion of the N-methyldiethanolamine was observed (¹H NMR). The Na₂MIDA (II a.3) was obtained with a selectivity above 90% (¹H NMR).

### II.4 Oxidation of compound (I a.1) to MGDA-Na₃

Reactor 1 was charged with a suspension of Pt,Bi/C (12.83 g, 1.38 mmol, 2.5 mol%, 4.97 wt.-% Pt (dry), 42 wt.-% in H₂O, molar ratio, Pt : Bi = 3.57 : 1) in water, and cooled to 15°C. Then, an aqueous solution of the sodium salt of (I a.1) (17.3 g, 55.2 mmol, 1 eq., 63.6 wt.-% in H₂O) was added to a (161 mL). Sodium hydroxide (9.72 g, 121.5 mmol, 2.2eq, 50 wt.-% in H₂O) was then added over a period of 10 minutes. The inner temperature control was set to 20°C. At 20°C, oxygen (20 NL/h) was bubbled in the reaction through a glass frit. After 7 hours, the oxygen flow was stopped, and the catalyst was removed by filtration. Full conversion of compound (I a.1) was observed (¹H NMR). The molar ratio of (II a.1) and (II b.1) (in each case, R1 = CH₃-CH(COOH) was 1.14:1 (determined by ¹H NMR). Further impurities were 1% by weight of sodium acetate and sodium salt of glycolic acid, 2 % by weight, both regarding to the sum of (II. a.1) and (II b.1) (determined by HPLC).

### III. Manufacture of detergent compositions

Preferred example automatic dishwashing formulations may be made according to Table 1.

**Table 1: Example detergent compositions for automatic dishwashing**

| All amounts in g/sample | ADW.1 | ADW.2 | ADW.3 |
|---|---|---|---|
| Mixture according to example II.1 | 30 | 22.5 | 15 |
| protease | 2.5 | 2.5 | 2.5 |
| amylase | 1 | 1 | 1 |
| n-C₁₈H₃₇-O(CH₂CH₂O)₉H | 5 | 5 | 5 |
| Polyacrylic acid M_{w} 4000 g/mol as | 10 | 10 | 10 |
| sodium salt, completely neutralized | | | |
| Sodium percarbonate | 10.5 | 10.5 | 10.5 |
| TAED | 4 | 4 | 4 |
| Na₂Si₂O₅ | 2 | 2 | 2 |
| Na₂CO₃ | 19.5 | 19.5 | 19.5 |
| Sodium citrate dihydrate | 15 | 22.5 | 30 |
| HEDP | 0.5 | 0.5 | 0.5 |
| ethoxylated polyethylenimine, 20 EO/NH group, Mₙ: 30,000 g/mol | optionally: 0.1 | optionally: 0.1 | optionally: 0.1 |

With the exemplified automatic dishwashing compositions, excellent dishwashing results are obtained.

## Claims

1. Process for the synthesis of compounds with the general formula (II) or their respective alkali metal salts by oxidation of compounds of general formula (I) with oxygen 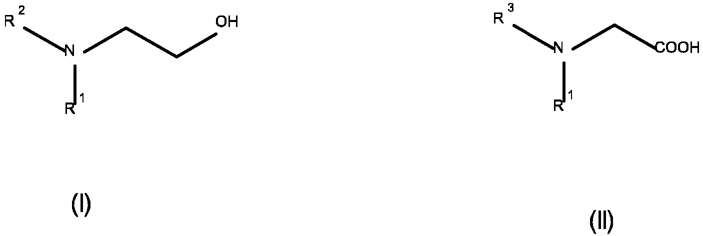
in the presence of a heterogeneous catalyst containing a metal of group 10 of the periodic table of the elements,
wherein R¹ and R² and R³ are same or different and selected from H and C₁-C₆-alkyl, non-substituted or substituted with one or more moieties selected from COOH and O-R⁴ with R⁴ being selected from H and C₁-C₄-alkyl, wherein the catalytically active metal is Pt and wherein at least one promotor selected from Cd, Tl, Pb and Bi is present in the heterogeneous catalyst, and wherein the oxidation is carried out at a temperature in the range of from -10 to +40°C.

2. Process according to claim 1 wherein the oxidation is carried out in an aqueous medium.

3. Process according to any of the previous claims wherein the reaction mixture contains at least 0.9 mol of a base per mol of hydroxyl group in compound of general formula (I).

4. Process according to any of the previous claims wherein the catalytically active metal is deposited on a support.

5. Process according to any of the previous claims wherein the partial pressure of oxygen during the oxidation is in the range of from 0.05 to 8 bar.

6. Process according to any of the preceding claims wherein R¹ is CH₃-CH(COOH) or HOOC-(CH₂)₂-CH-COOH, as free acid or at least partially neutralized with alkali.

7. Process according to any of the preceding claims wherein compound (I) bears an impurity of from 0.01 to 10% by weight of glycols, referring to compound (I).

8. Process according to any of the previous claims where the starting material is a compound according to general formula (I a) 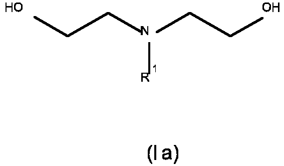 wherein R¹ is defined as above.

9. Process according to any of the previous claims where the reaction product essentially comprises of compounds according to general formulae (II a) or (II b) 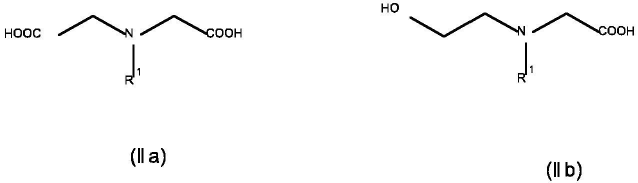 or the respective alkali metal salts wherein R¹ is defined as above.

10. Process according to claim 9 wherein the reaction product essentially comprises compounds according to general formulae (II a) and (II b) in a molar ratio in the range of from 20:1 to 1:1 and in the range of from 2 to 15% by weight glycolic acid, referring to the sum of compounds according to general formulae (II a) and (II b).

11. Mixture that comprises compounds according to general formulae (II a) and (II b) 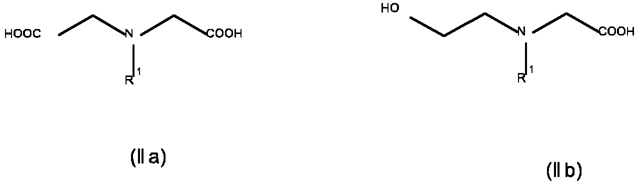
or their respective alkali metal salts, in a molar ratio in the range of from 2 to 15% by weight glycolic acid, referring to the sum of compounds according to general formulae (II a) and (II b), and less than 0.1% by weight of oxalic acid, or their respective alkali metal salts,
wherein R¹ is selected from H and C₁-C₆-alkyl, non-substituted or substituted with one or more moieties selected from COOH and O-R⁴, with R⁴ being selected from H and C₁-C₄-alkyl
and wherein the percentages refer to the respective sodium salts.

12. Mixture according to claim 11 wherein R¹ is CH₃-CH(COOH) or HOOC-(CH₂)₂-CH-COOH, optionally partially or fully neutralized with alkali.

## Patentansprüche

1. Verfahren zur Synthese von Verbindungen mit der allgemeinen Formel (II) oder deren jeweiligen Alkalimetallsalzen durch Oxidation von Verbindungen der allgemeinen Formel (I) mit Sauerstoff 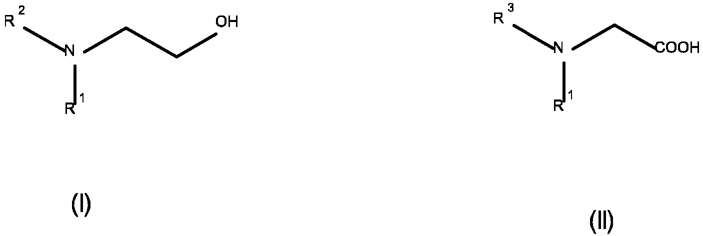
in Gegenwart eines heterogenen Katalysators, der ein Metall der Gruppe 10 des Periodensystems der Elemente enthält,
wobei R¹ und R² und R³ gleich oder verschieden sind und aus H und C₁-C₆-Alkyl, das unsubstituiert oder durch eine oder mehrere Gruppierungen, die aus COOH und O-R⁴ ausgewählt sind, substituiert ist, ausgewählt sind, wobei R⁴ aus H und C₁-C₄-Alkyl ausgewählt ist, wobei es sich bei dem katalytisch aktiven Metall um Pt handelt und wobei mindestens ein aus Cd, Tl, Pb und Bi ausgewählter Promotor in dem heterogenen Katalysator vorliegt, und wobei die Oxidation bei einer Temperatur im Bereich von -10 bis +40 °C durchgeführt wird.

2. Verfahren nach Anspruch 1, wobei die Oxidation in einem wässrigen Medium durchgeführt wird.

3. Verfahren nach einem der vorhergehenden Ansprüche, wobei die Reaktionsmischung mindestens 0,9 mol einer Base pro Mol Hydroxylgruppen in der Verbindung der allgemeinen Formel (I) enthält.

4. Verfahren nach einem der vorhergehenden Ansprüche, wobei das katalytisch aktive Metall auf einem Träger abgeschieden ist.

5. Verfahren nach einem der vorhergehenden Ansprüche, wobei der Sauerstoffpartialdruck während der Oxidation im Bereich von 0,05 bis 8 bar liegt.

6. Verfahren nach einem der vorhergehenden Ansprüche, wobei R¹ für CH₃-CH (COOH) oder HOOC-(CH₂)₂-CH-COOH, als freie Säure oder zumindest teilweise mit Alkali neutralisiert, steht.

7. Verfahren nach einem der vorhergehenden Ansprüche, wobei Verbindung (I) eine Verunreinigung von 0,01 bis 10 Gew.-% Glykolen, bezogen auf Verbindung (I), trägt.

8. Verfahren nach einem der vorhergehenden Ansprüche, wobei es sich bei dem Ausgangsstoff um eine Verbindung gemäß der allgemeinen Formel (I a) 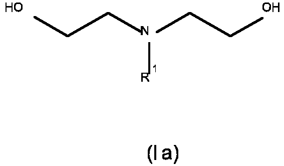 handelt, wobei R¹ wie oben definiert ist.

9. Verfahren nach einem der vorhergehenden Ansprüche, wobei das Reaktionsprodukt im Wesentlichen Verbindungen gemäß den allgemeinen Formeln (II a) oder (II b) 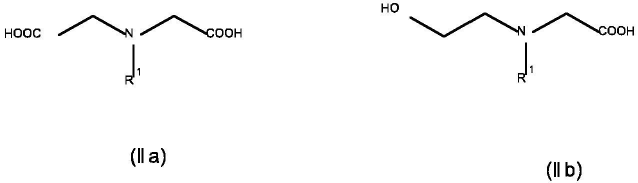 oder den jeweiligen Alkalimetallsalzen umfasst, wobei R¹ wie oben definiert ist.

10. Verfahren nach Anspruch 9, wobei das Reaktionsprodukt im Wesentlichen Verbindungen gemäß den allgemeinen Formeln (II a) und (II b) in einem Molverhältnis im Bereich von 20:1 bis 1:1 und im Bereich von 2 bis 15 Gew.-% Glykolsäure, bezogen auf die Summe von Verbindungen gemäß den allgemeinen Formeln (II a) und (II b), umfasst.

11. Gemisch, das Verbindungen der allgemeinen Formeln (II a) und (II b) 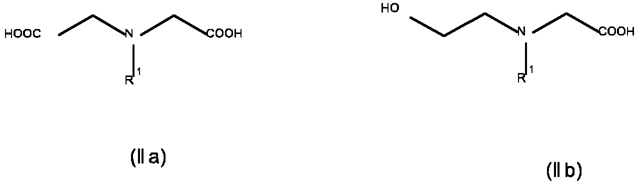
oder deren jeweilige Alkalimetallsalze in einem Molverhältnis im Bereich von 2 bis 15 Gew.-% Glykolsäure, bezogen auf die Summe von Verbindungen gemäß den allgemeinen Formeln (II a) und (II b), und weniger als 0,1 Gew.-% an Oxalsäure oder deren jeweiligen Alkalimetallsalzen umfasst,
wobei R¹ aus H und C₁-C₆-Alkyl, das unsubstituiert oder durch eine oder mehrere Gruppierungen, die aus COOH und O-R⁴ ausgewählt sind, substituiert ist, ausgewählt ist, wobei R⁴ aus H und C₁-C₄-Alkyl ausgewählt ist,
und wobei sich die Prozentangaben auf die jeweiligen Natriumsalze beziehen.

12. Gemisch nach Anspruch 11, wobei R¹ für CH₃-CH(COOH) oder HOOC-(CH₂)₂-CH-COOH, gegebenenfalls teilweise oder vollständig mit Alkali neutralisiert, steht.

## Revendications

1. Procédé pour la synthèse de composés de formule générale (II) ou de leurs sels de métaux alcalins respectifs par oxydation de composés de formule générale (I) avec de l'oxygène 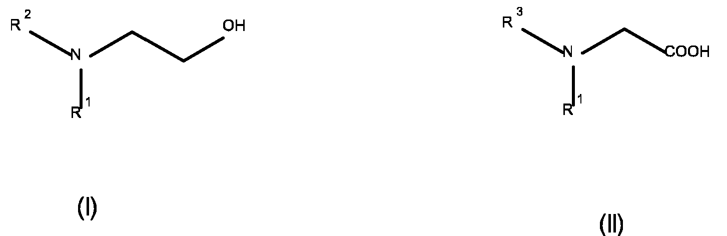
en présence d'un catalyseur hétérogène contenant un métal du groupe 10 du tableau périodique des éléments,
R¹ et R² et R³ étant identiques ou différents et choisis parmi H et C₁-C₆-alkyle, non substitué ou substitué par un ou plusieurs groupements choisis parmi COOH et O-R⁴,
R⁴ étant choisi parmi H et C₁-C₄-alkyle, le métal catalytiquement actif étant Pt et au moins un promoteur choisi parmi Cd, Tl, Pb et Bi étant présent dans le catalyseur hétérogène, et dans lequel l'oxydation est effectuée à une température comprise dans la plage de - 10 à +40 °C.

2. Procédé selon la revendication 1, dans lequel l'oxydation est effectuée dans un milieu aqueux.

3. Procédé selon l'une quelconque des revendications précédentes, dans lequel le mélange réactionnel contient au moins 0,9 mole d'une base par mole de groupe hydroxyle dans le composé de formule générale (I).

4. Procédé selon l'une quelconque des revendications précédentes, dans lequel le métal catalytiquement actif est déposé sur un support.

5. Procédé selon l'une quelconque des revendications précédentes, dans lequel la pression partielle d'oxygène pendant l'oxydation est dans la plage de 0,05 à 8 bars.

6. Procédé selon l'une quelconque des revendications précédentes, dans lequel R¹ est CH₃-CH(COOH) ou HOOC-(CH₂)₂-CH-COOH, sous forme d'acide libre ou au moins partiellement neutralisé avec un alcali.

7. Procédé selon l'une quelconque des revendications précédentes, dans lequel le composé (I) porte une impureté de 0,01 à 10 % en poids de glycols, par rapport au composé (I).

8. Procédé selon l'une quelconque des revendications précédentes, dans lequel la matière première est un composé selon la formule générale (I a) 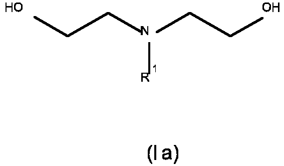 dans lequel R¹ est comme défini ci-dessus.

9. Procédé selon l'une quelconque des revendications précédentes, dans lequel le produit de réaction est essentiellement composé de composés selon les formules générales (II a) ou (II b) 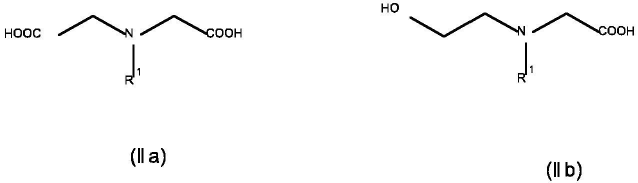 ou les sels de métaux alcalins respectifs, dans lequel R¹ est comme défini ci-dessus.

10. Procédé selon la revendication 9, dans lequel le produit de réaction est essentiellement composé de composés selon les formules générales (II a) et (II b) en un rapport molaire dans la plage de 20:1 à 1:1 et dans la plage de 2 à 15 % en poids d'acide glycolique, par rapport à la somme des composés selon les formules générales (II a) et (II b).

11. Mélange qui comprend des composés selon les formules générales (II a) et (II b) 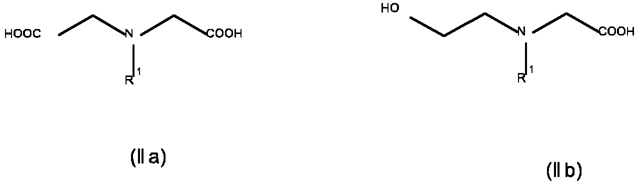
ou leurs sels de métaux alcalins respectifs, en un rapport molaire dans la plage de 2 à 15 % en poids d'acide glycolique, par rapport à la somme des composés selon les formules générales (II a) et (II b), et moins de 0,1 % en poids d'acide oxalique, ou de leurs sels de métaux alcalins respectifs,
dans lequel R¹ est choisi parmi H et C₁-C₆ alkyle, non substitué ou substitué par un ou plusieurs groupements choisis parmi COOH et O-R⁴, R⁴ étant choisi parmi H et C₁-C₄ alkyle
et, les pourcentages faisant référence aux sels de sodium respectifs.

12. Mélange selon la revendication 11, dans lequel R¹ est CH₃-CH(COOH) ou HOOC-(CH₂)₂-CH-COOH, éventuellement partiellement ou totalement neutralisé avec un alcali.
